# EUROPEAN PATENT SPECIFICATION

(11) **EP 2 012 660 B1**
(45) Date of publication and mention of the grant of the patent: **02.09.2009**
(21) Application number: 07736229.1
(22) Date of filing: 18.04.2007
(51) Int. Cl.: A61B 5/021

(54) **Apparatus for controlled blood regurgitation through tricuspid valve**
Gerät zur kontrollierten Blutregurgitation durch die Trikuspidalklappe
Appareil pour la régurgitation régulée de sang par l'intermédiare d'une valvule tricuspide

(30) Priority: 19.04.2006 US 792905 P
(43) Date of publication of application: 14.01.2009
(73) Proprietor: Transpid Ltd., 38900 Caesarea (IL)
(72) Inventor: ROTTENBERG, Dan, 34601 Haifa (IL)
(74) Representative: Minoja, Fabrizio
(86) International application number: PCT/IL2007/000489
(87) International publication number: WO 2007/119241

(56) References cited:
- EP-A- 0 363 117
- US-A- 3 995 623
- US-A- 4 889 137
- US-A- 5 509 428
- US-A- 5 738 652

## Description

### FIELD OF THE INVENTION

This invention relates to a catheter assembly for inducement and monitoring of a tricuspid regurgitation for the treatment of different heart failure conditions such as lung edema.

### BACKGROUND OF THE INVENTION

Congestive Heart Failure (CHF) is a condition characterized by the inability of the heart to pump blood at a rate needed to maintain adequate blood flow throughout the body. CHF most commonly occurs as a result of ischemic heart disease, myocardial infarction, cardiomyopathy, or valvular disorders. In most cases, the left ventricle (which pumps oxygenated blood to the body) fails, while the right ventricle (which pumps deoxygenated blood to the lungs) continues to function normally. When this occurs, the pulmonary arterial pressures increase and fluid begins to exude from the pulmonary capillaries into the pulmonary interstitium and eventually into the alveoli, the tiny air sacs where gas exchange takes place. Fluid in the alveoli impedes gas exchange across the alveolar membranes, which then lowers the blood oxygenation. The resulting hypoxemia has a detrimental effect on the cardiac muscle, which requires large amounts of oxygenated blood to function normally, and results in an even more profound heart failure. This cycle, if left untreated, continues until the patient succumbs, in effect, drowning in his own pulmonary fluids.

During the course of CHF induced lung edema, some patients develop a condition named tricuspid regurgitation, whereby a faulty valve allows blood to reflux backward through the valve into the right atrium. It has been found that those patients who develop tricuspid regurgitation during the course of CHF actually experience improvements in their pulmonary symptoms. Such edema improvement can theoretically be achieved without any significant reduction in cardiac output, if regurgitation level and reduction of pulmonary pressures are controlled, and not reduced below certain levels that effect left ventricle cardiac output (known in the art as Frank-Sterling curve).

Several types of catheters and methods for inserting catheters through the tricuspid valve are disclosed in the prior art.

U.S. Pat. No. 5,207,228 discloses a catheter for monitoring heart function, which comprises a catheter tube having a plurality of lumens. The catheter includes an inflatable balloon at a distal tip of the catheter tube for positioning the catheter in a wedged position within a pulmonary artery within the heart of a patient. Dual injectate ports are formed in a side wall of the catheter tube. Each port communicates with a respective injectate lumen carried in the catheter tube. When inserted into the heart of a patient, either the first or second injectate port is positioned within the desired distance from the tricuspid valve for thermodilution depending upon the size of the heart. Thermodilution to obtain cardiac output and/or right heart ejection fraction is implemented by injecting injectate through the lumen associated with the port properly positioned relative the tricuspid valve and discharged through the corresponding port.

In U.S. Pat. No. 5,509,428 a method and apparatus are disclosed for the controlled inducement of tricuspid regurgitation for the purpose of treating congestive heart failure. The apparatus is comprised of a catheter onto which is affixed an expandable device. The catheter is percutaneously inserted into the patient's body, and then moved within the central venous system until the end of the catheter traverses the tricuspid valve of the heart. The expandable device on the distal end of the catheter is then radially expanded to hold open the leaflets of the tricuspid valve. The catheter advancement and positioning as disclosed in this patent is performed through fluoroscopic observation.

The EP0363117 discloses a balloon-tipped, flow-directed catheter with a position-monitoring port is adapted to be positioned in the outflow tract of the right ventricle proximal to the pulmonic valve of the heart, which in most adult patients would be approximately 8 cm from the distal tip of the catheter. By measuring the pressure at the position-monitoring port, the position of the catheter tip and the balloon in the pulmonary artery can be monitored, and checked before inflating the balloon to measure pulmonary wedge pressure at the distal port. A right ventricular pressure reading indicates that the balloon is in a proper position in the pulmonary artery for measuring wedge pressure. A pulmonary artery pressure reading indicates that the position-monitoring port has crossed the pulmonic valve and that the balloon has migrated distally to a position that may not be safe for inflating the balloon to measure wedge pressure. In such a case, the catheter should be gradually withdrawn until a right ventricular pressure is obtained.

U.S. Pat. No. 5,034,001 discloses a vascular catheter having an expandable cage mounted on the distal end of a tubular member which is radially expanded and contracted by means of a control wire which is secured to the distal end of the expandable cage. The control wire extends through a first inner lumen within the tubular member which extends along essentially the entire length thereof. A second inner lumen is provided in the distal portion of the tubular member which has a proximal port at least 15 but not more than 60 cm from the distal end of the catheter and a distal port which opens into the interior of the expandable cage. A guidewire or a low-profile steerable catheter is slidably disposed within the second lumen and a tubular member such as a slightly expanded coil through the expandable cage interior to facilitate the rapid exchange of the catheter. The catheter assembly is particularly adapted to hold open a blood vessel after a vascular procedure therein such as an angioplasty.

A catheter which includes means to measure local pressure at two or more points along the catheter body is described in WO0113789. The points are preferably located in two different pressure areas, more preferably across a valve in a vessel, organ or similar.

### SUMMARY OF THE INVENTION

The present invention is concerned with a device for precise positioning of an expander-catheter system used for inducing controllable tricuspid regurgitation. Using the catheter facilitates *in-situ* position confirmation without the need for radioscopic or other observation and thus simplifies cardiac procedures.

According to a first aspect of the present invention there is provided a catheter assembly comprising a catheter tube having a proximal end and a distal end, configured to pass axially through a venous system, said distal end fitted with a navigation balloon inflatable through an inflation lumen partially extending through a major portion of the tube; an expander surrounding a section of said catheter, extending between a rear end and a fore end, and having a blood flow path, the assembly further comprising a manipulating sleeve having a front end and a rear end slidingly enveloping a portion of the tube extending rearwards from the expander and forming a gap between the tube and said sleeve, whereby said expander is adapted to radially expand and contract through manipulation of the sleeve; the tube further comprising a first pressure lumen extending from the distal end for measuring a first pressure point, a second pressure lumen extending from adjacent in front of the fore end of the expander for measuring a second pressure point and a third pressure conduit extending from adjacent the fore end of the expander for measuring a third pressure point, said first and second pressure lumens and the third pressure conduit terminate at the tubes proximal end at pressure outlet couplings.

According to one embodiment of the present invention the expander normally extends flush over the tube and its fore end is secured over the tube and its rear end is slidably displaceable thereon; and where a front end of the manipulating sleeve is engaged with a rear end of the expander to thereby axially displace said rear end of the expander so as to facilitate its expansion and contraction.

According to another embodiment the expander is normally biased to spontaneously expand over the tube, where its fore end and its rear end are affixed over the tube and whereby the manipulating sleeve extends over the expander and retains it at its retracted configuration whereby upon retraction of the sleeve the expander is gradually exposed to thereby expand its exposed portion.

### BRIEF DESCRIPTION OF THE DRAWINGS

In order to understand the invention , embodiments will now be described, by way of non-limiting examples only, with reference to the accompanying drawings, in which:
**Fig. 1A** is a general view of a cardiac catheter according to an embodiment of the present invention
**Fig. 1B** is a schematic longitudinal section of the catheter assembly according to one embodiment of the present invention;
**Fig. 1C** is a cross section along line C-C in Fig. 1A;
**Fig. 1D** is a cross section along line D-D in Fig. 1A;
**Fig. 1E** is a cross section along line E-E in Fig. 1A.
**Fig. 2** is a schematic longitudinal section of a catheter assembly according to another embodiment of the present invention;
**Figs. 3A** and **3B** illustrate the expander portion of the catheter assembly according to Fig. 2 in a collapsed and the expanded configurations respectively;
**Figs. 4A** to **4C** illustrate consecutive steps of expanding the expander of the catheter assembly between a collapsed position (Fig. 4A) and fully expanded position (Fig. 4C);
**Fig. 5** is a schematic representation of a heart fitted with the catheter assembly of the present invention; with the expander at its collapsed configuration;
**Fig. 6** is similar to Fig. 5 wherein the catheter assembly is in its expanded configuration; and
**Figs. 7A** to **7C** are a waveform representation of the pressure measurements as received from ports P_{I}, P_{II} and P_{III}, respectively.
**Fig. 8** is a schematic illustration of an expander according to an embodiment of the present invention.

### DETAILED DESCRIPTION OF EMBODIMENTS

The present invention discloses an apparatus for controlled inducement of tricuspid regurgitation for the purpose of treating congestive heart failure.

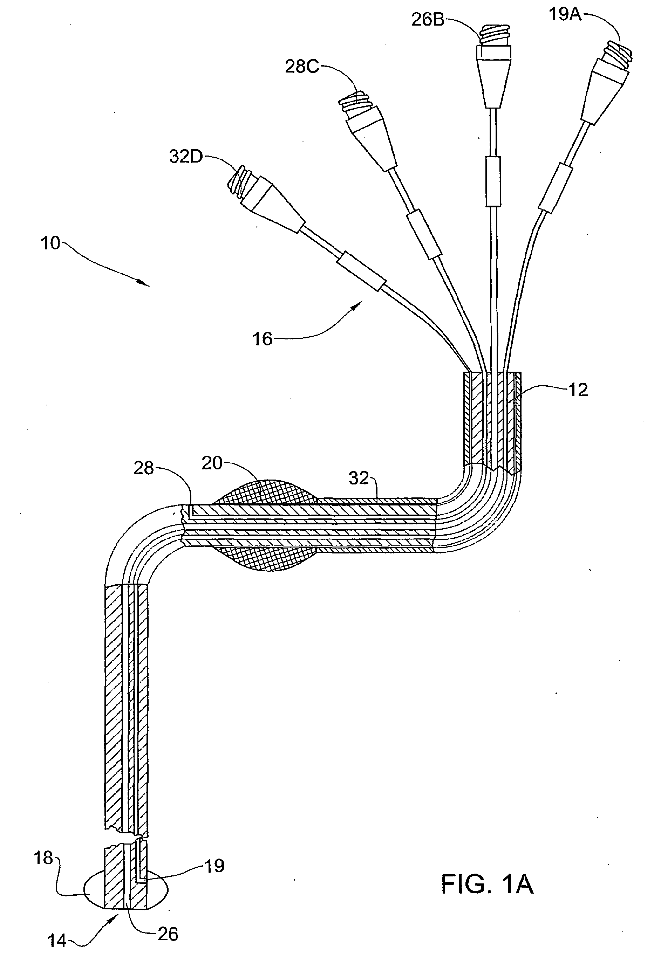

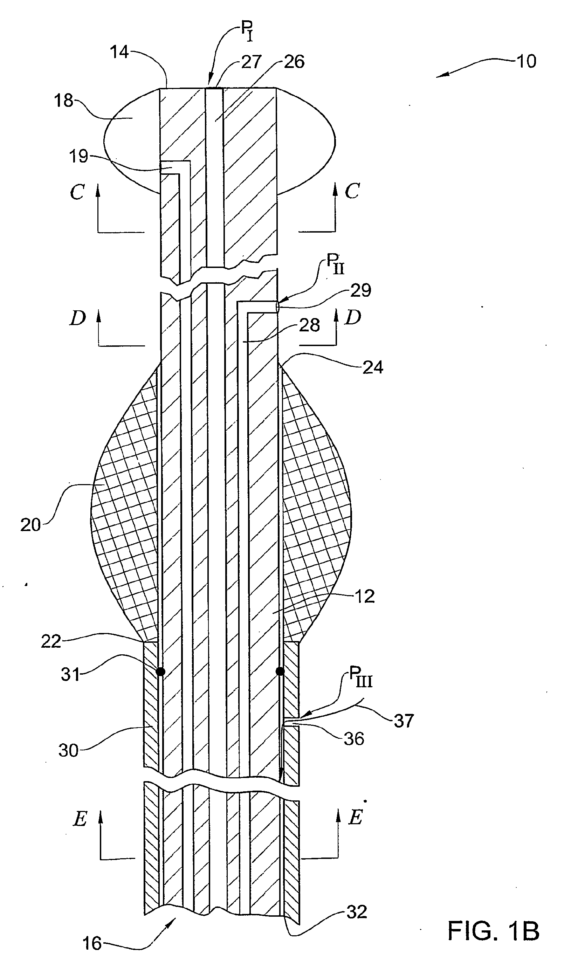

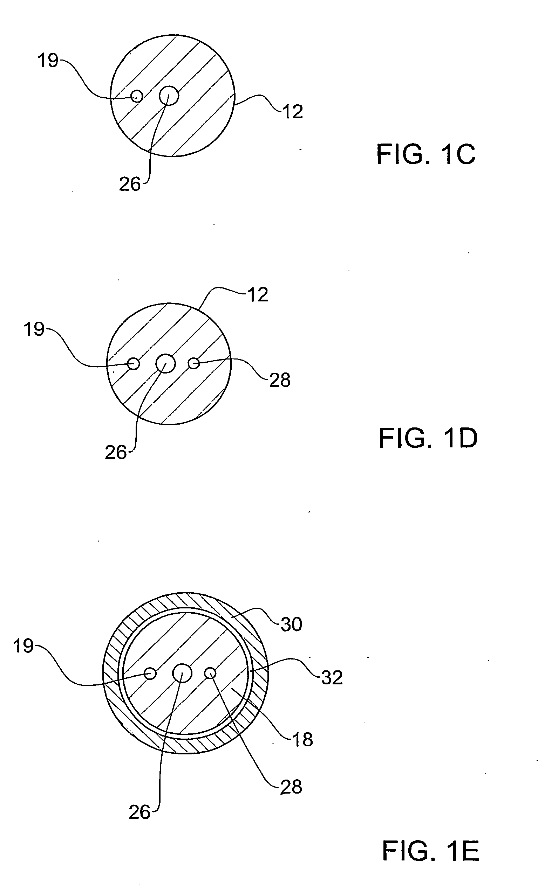

Attention is first directed to Figs. 1A and 1B of the drawings, illustrating a catheter assembly generally designated **10,** in accordance with one embodiment of the present invention, comprising, a tube **12** having a proximal end **16** and a distal end **14,** said catheter assembly configured to pass axially through the venous system and right ventricle. The distal end **14** of the catheter tube **12** is fitted with a navigation balloon **18** inflatable through an inflation lumen **19** extending through a major portion of the catheter tube **12.**

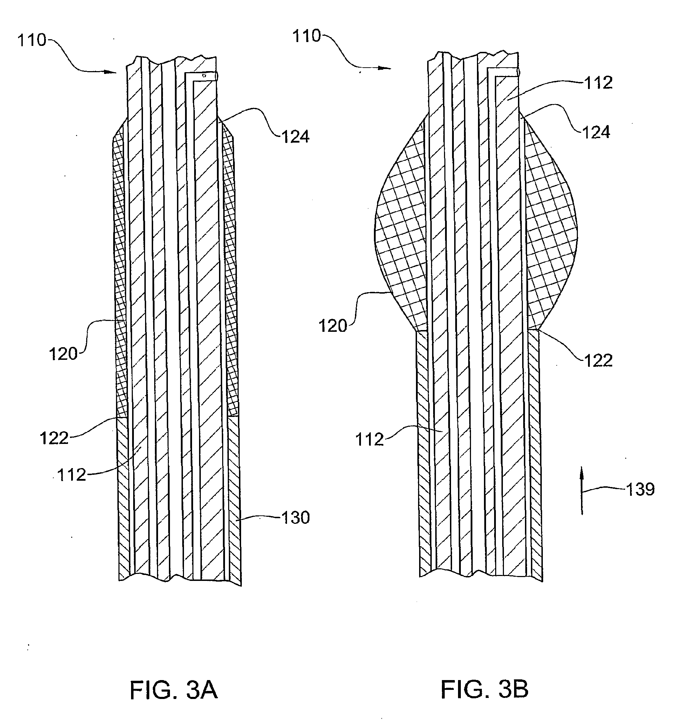

The catheter assembly **10** of the present invention further comprises an expander **20** surrounding a section of said catheter tube **12** and extending between a rear end **22** and a fore end **24,** the expander **20** adapted to allow blood flow therethrough. According to the present embodiment, at its contracted configuration (see Fig. 3A), the expander **20** extends substantially flash over the tube **12** where its fore end **24** is secured over the tube **12** and its rear end **22** is slidably displaceable over the tube. The rear end **22** of the expander **20** is engaged with a manipulating sleeve **30** slidingly enveloping a portion of the tube **12** extending rearwards from said rear end **22** whereby the sleeve **30** is adapted to displace said rear end **22** of the expander **20** so as to facilitate expansion and contraction of the expander **20** (Figs. 3A and 3B, respectively). The sleeve **30** envelopes the tube such that a gap **32** is formed between the tube **12** and said sleeve **30,** said gap facilitating a third pressure conduit **32** as will be discussed hereinafter.

The catheter tube **12** further comprises a first pressure lumen **26** extending from or adjacent the distal end **14** towards the proximal end **16,** adapted to measure a first pressure point referred to as **P_{I}** through a first pressure port **27.** A second pressure lumen **28** extends from adjacent in front of the fore end **24** of the expander **20** towards the proximal end **16,** adapted to measure a second pressure point referred to as **P_{II}** through a second pressure port **29.**

According to this embodiment, the manipulating sleeve **30** is fitted with a sealing ring **31** proximal the rear end **22** of the expander **20,** thus preventing blood flow through the expander **20** into the gap **32.**

The third pressure conduit **32** extends between the sealing ring **31** towards the proximal end **16** with an inlet port **36** for measuring a third pressure point **P_{III}.** The inlet port **36** moves with the sleeve **30** when pulled or pushed relative to tube **12** (the inlet port displaces axially along the tube).

Attention is also directed to Figs. 1C through 1E illustrating respective sections taken along the catheter **10** and schematically illustrating the configuration of the catheter.

Each of the pressure lumens **26, 28,** and the third pressure conduit **32** extend towards pressure outlet coupling fitted at the proximal **16** of the catheter **10** fitted for connection to a pressure monitor, as will be discussed hereinafter. Likewise, the inflation lumen **19** extends towards an inflating port at the proximal end of the catheter assembly **16** for coupling to a pressure source, e.g. syringe (not shown). The lumens **19, 26, 28** and the pressure conduit 32 communicate with proximal end connector tubes **19A, 26B, 28C** and **32D,** respectively, as shown in Fig. 1A. Tube **19A** leads to connector (not shown) for connection to an inflation apparatus, such as an inflation syringe. Bacteria-filtered carbon dioxide is one type of inflation medium beneficial due to its rapid absorption in the blood in the event of balloon rupture within the circulation.

Tubes **26B, 28C** and **32D** connect to a pressure measurement apparatus (not shown) in order to monitor pressures at the pressure ports **27, 29** and **36.**

For sake of clarity, further embodiments of the present invention will be discussed with reference to figures, wherein like components are designated with like numerals shifted by hundred.

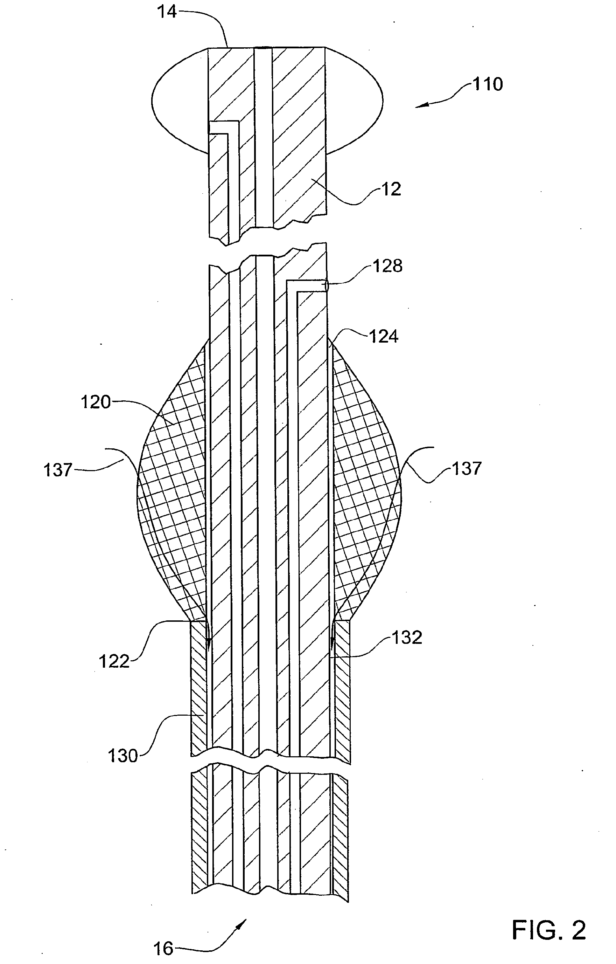

Fig. 2 is another embodiment of a catheter assembly generally designated **110,** substantially similar to the embodiment of Figs. 1A-1E, however, with a fore end **124** of the expander **120** affixed to a tube **112** and whereby a manipulating sleeve **130** is engaged with the rear end **122** of the expander **120,** such that a blood flow path (illustrated by arrow **137)** extends through the expander **120** and a third pressure conduit **132** for measurement of the third pressure point **P_{III}.**

In Figs. 3A and 3B a partial illustration of the catheter assembly **110** of Fig. 2 is shown. As can be seen in Fig. 3A the expander **120** is in its contracted configuration fitted substantially flash over the tube **112** with its fore end **124** secured to the tube **112** and its rear end **122** engaged with the manipulating sleeve **130,** whereby in Fig. 3B the manipulating sleeve **130** has been pushed forwards in direction of arrow **139** with corresponding axial displacement of the rear end **122** (whilst its fore end **124** is fixed over the tube **112),** such that the expander **120** radially expands. It is appreciated that the extent of radial expansion depends on an amount of the axial displacement of the rear end **122.**

The expander **20, 120** according to the present invention can be a wire mesh or any other suitable configuration fitted for controlled radial expansion whilst allowing fluid flow therethrough at its expanded configuration. The expander may be made of metal wire, fabric, plastic material etc. the expander, according to any of its embodiments may be made of or coated by a bio-compatible material.

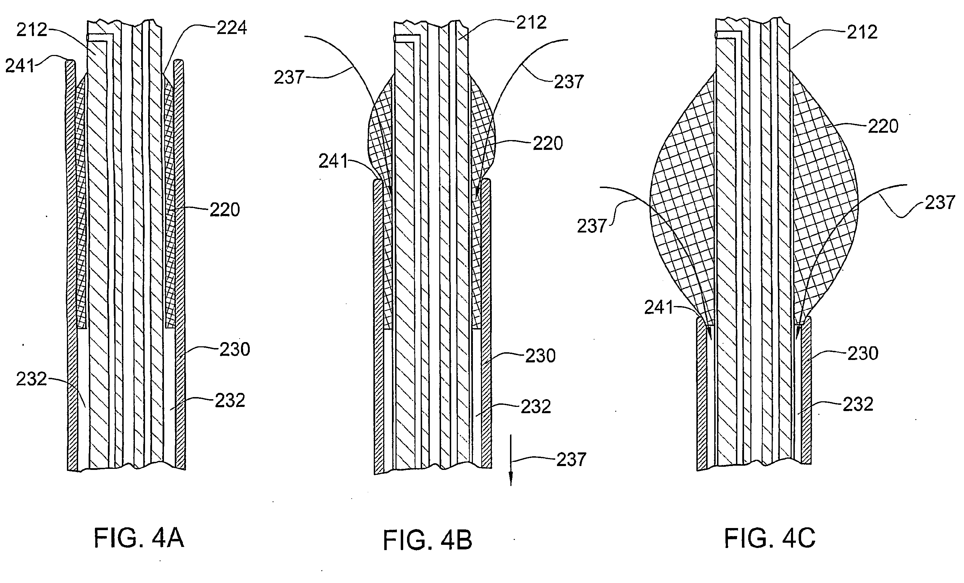

In Figs. 4A to 4C an additional embodiment of the present invention is illustrated, this embodiment is substantially similar to the previous embodiments, wherein the main differences reside in the expander and the expansion mechanism as will be discusses hereinafter. The configuration of the pressure lumens is substantially similar to the previous embodiment as already discussed.

As can be seen in Fig. 4A, both the fore end **224** and the rear end **222** of an expander **220** are secured over a tube **212.** The expander **220** is made of memory shape alloy and is normally biased into spontaneous expansion. However, a manipulating sleeve **230** embraces the expander **220** such that it prevents spontaneous expansion thereof. The manipulating sleeve **230** is slidingly displaceable over the tube **212.** Upon axial withdrawal of the sleeve **230** in a direction of the arrow **237** (Fig. 4B), the expander **220, begins** to spontaneously expand until a maximum expansion is achieved upon complete retraction of manipulating sleeve **230** (Fig. 4C)

According to this embodiment a third pressure conduit **232** extends from the fore end **241** of the manipulating sleeve **230,** though the expander **220** to the proximal end **216** (not seen) of the catheter assembly **210** such that the blood enters the gap **232** between the manipulating sleeve **230** and the tube **212.**

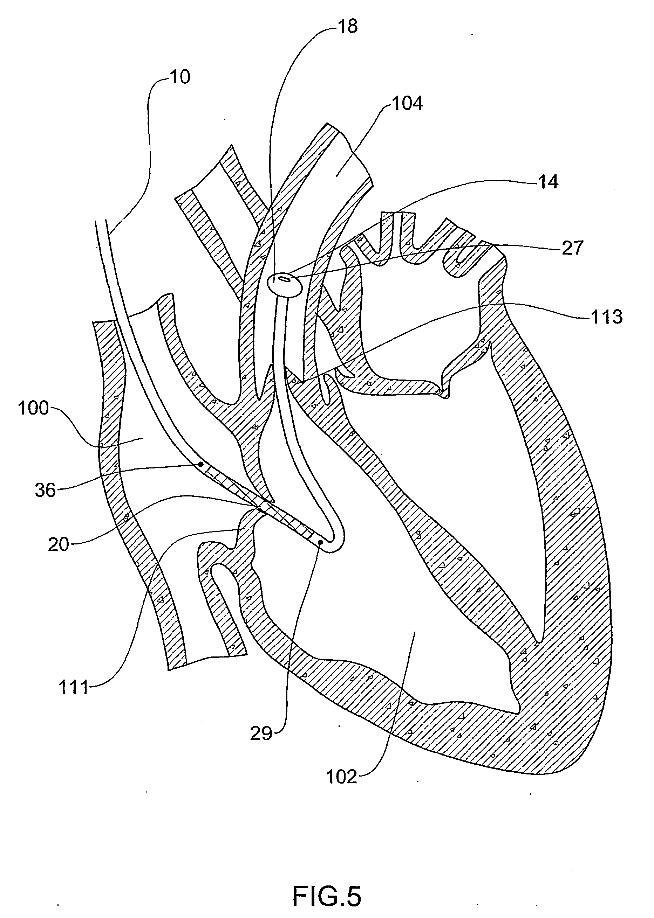

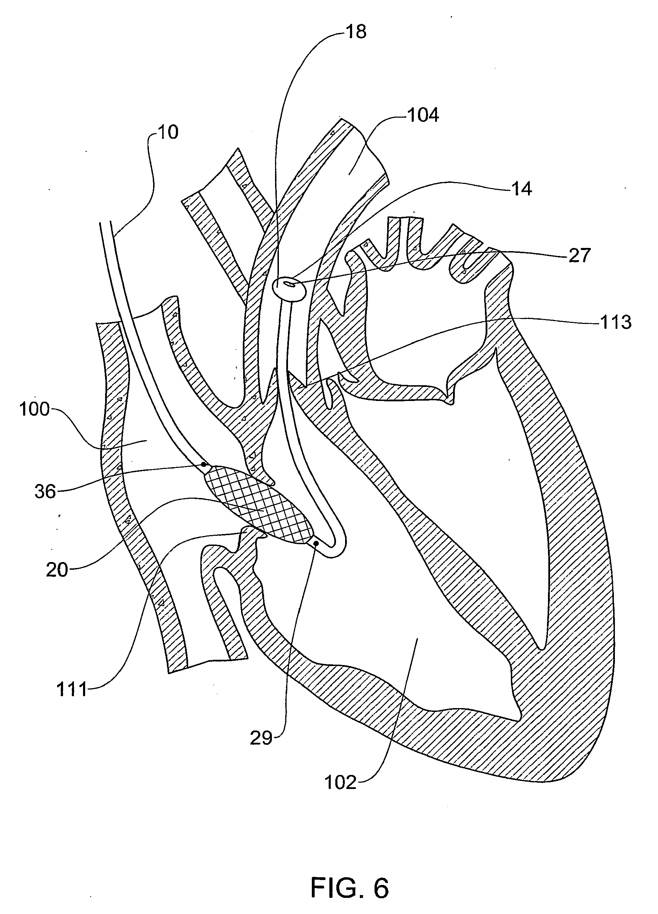

Figs. 5 and 6 illustrate a particular method for utilizing the catheter assembly , for controlled inducement of the tricuspid regurgitation. The method is exemplified with reference to Figs. 1A-3B, though it is applicable using a catheter assembly according to any of other embodiments as well.

The catheter assembly **10** is initially inserted into the patient's venous system with the expander **20** at its contracted configuration and with the balloon **18** deflated (not shown). The assembly **10** can be introduced through a large vein, often the internal jugular, subclavian, or femoral veins.

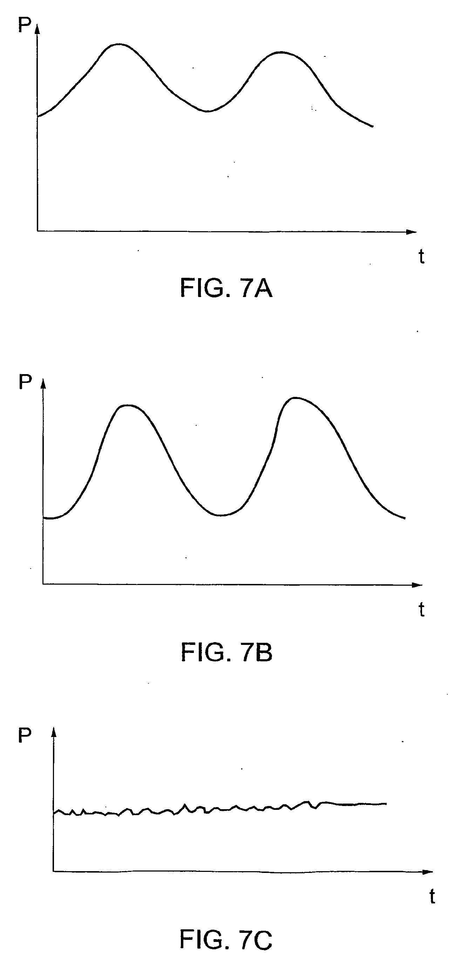

The catheter assembly **10** is advanced through the right atrium **100.** Once within the right atrium **100** the flow-directed navigation balloon **18** is inflated through the inflation lumen **19** (e.g. by aid of predetermined volume syringe). Hydraulics of the blood flow advance the inflated balloon **18** through the tricuspid valve **111** into the right ventricle **102** and thus carries and navigates the distal end **14** of the catheter assembly **10** through the pulmonary valve **113** and into the main pulmonary artery **104.** The position of the balloon **18** within the pulmonary artery **104** is confirmed through monitoring pressure point **P_{I}** through pressure lumen **26** (yielding a pressure graph as in Fig. 7A).

It is now required to position the expander **20** within the vicinity of the tricuspid valve **111** for efficient and controlled expansion thereof.

Additional pressure measurements are now taken through the second pressure lumen **28** and third pressure conduit **32** to confirm positioning of the expander **20** at its desired position within the tricuspid valve **111.** The position of the expander **20** is confirmed through measurement of pressure points **P_{II}** and **P_{III},** wherein **P_{II}** indicates the pressure in the right ventricle (Fig. 7B) and **P_{III}** indicates the pressure at the right atrium (Fig. 7C). The pressure ports **29** and **36** are respectively positioned adjacent before and behind the expander **20,** at all times (i.e. at its collapsed and partially or fully expanded positions), thus providing definite pressure indicia corresponding with the pressure upstream and downstream the valve **111,** confirming that the expander **20** is positioned within the tricuspid valve **111.**

In case pressure measurements obtained at the second pressure measurement point **P_{II}** and the third measurement point **P_{III}** do not fall within the expected values of the pressure measurements of the right ventricle **102** and right atrium **100** (Figs. 7B and 7C, respectively), the catheter assembly **10** may easily be slightly advanced or retracted in order to obtain correct positioning confirmed by the respective pressure measurements.

Once the desired position for the expander **20** has been obtained, the balloon **18** may be deflated.

After correct position of the expander **20** within the valve **111** has been confirmed, the expander **20** is slowly expanded to a desired rate to facilitate blood reflux backward through the valve **111** to the right atrium **100,** condition also known as tricuspid regurgitation. The intention of causing the regurgitation is to lower the pulmonary pressures elevated as a result of inability of the failed left heart to respond and compensate for acute changes in the cardiovascular system, such as after-load elevation.

This procedure can advantageously be performed whilst a torso of the patient is substantially upright (e.g. seated) this being a significant advantage with patients with sever lung edema, and further without the need in observations such as radiology or fluoroscopic observation etc. which often require undesired mobilization of the patient, availability of radiology equipment and specially trained staff.

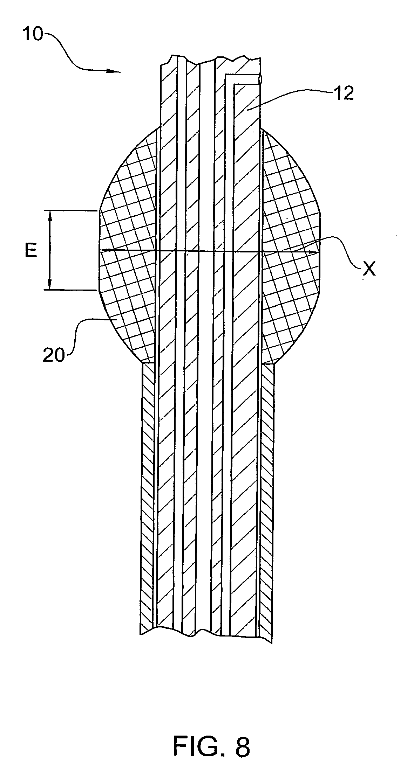

According to a particular embodiment of the present invention the length of the catheter assembly **10** is approximately 140 cm. The distance between the inflatable balloon **18** and the second pressure port **29** is in the range of 15-30 cm (according to a specific embodiment, it is 20 cm). The expander **20** according to the present invention in its contracted configuration is in the range of approximately 1 to 12 cm in length and wherein the length of an effective area for keeping the leaflets of the tricuspid valve in their open configuration is in the range of approximately 1 to 7 cm. The term "effective area" refers to an area having a mean diameter in the range of about 3-20 mm (see Fig. 8).

As can be seen in Fig. 6 after verifying pressure measurements to confirm correct positioning of the expander **20,** the expander is expanded. As described above this could be performed through various manipulating arrangements as discussed in connection with the embodiments hereinabove.

The rate of expansion of the expander **20** is monitored through measurements performed by pressure measuring ports **27, 29** and **36,** whereby the tricuspid valve **111** leaflets are held open and blood reflux is allowed from the right ventricle **102** into the right atrium **100** thus causing a tricuspid regurgitation. During this process patient's pulmonary arterial pressure and right ventricle pressure are closely monitored as well as the patients cardiac output. The degree of tricuspid regurgitation is thus controlled in order to obtain required pulmonary pressures

Whilst some embodiments have been described and illustrated with reference to some drawings, the artisan will appreciate that many variations are possible which do not depart from the general scope of the invention, *mutatis, mutandis.*

## Claims

1. A catheter assembly, comprising:
- a catheter tube (12) having a proximal end (16) and a distal end (14), configured to pass axially through a venous system and the right ventricle;
said distal end fitted with a navigation balloon (18) inflatable through an inflation lumen (19) partially extending through a major portion of the tube;
- an expander (20) surrounding a section of said catheter, extending between a rear end and a fore end, and having a blood flow path;
- a manipulating sleeve (30) having a front end and a rear end slidingly enveloping a portion of the tube extending rearwards from the expander and forming a gap between the tube and said sleeve, whereby said expander is adapted to radially expand and contract through manipulation of the sleeve;
- the tube further comprising a first pressure lumen (26) extending from the distal end for measuring a first pressure point through a first
pressure port (27);
a second pressure lumen (28) extending from adjacent in front of the fore end of the expander for measuring a second pressure point through a second pressure port (29); and
a third pressure conduit (32) extending from adjacent the rear end of the expander for measuring a third pressure point through an inlet port (36)
said first and second pressure lumens and the third pressure conduit terminate at the tubes proximal end at pressure outlet couplings.

2. A catheter assembly according to Claim 1, wherein the expander normally extends flush over the tube and where its fore end is secured over the tube and its rear end is slidably displaceable thereon; and where a front end of the manipulating sleeve is engaged with a rear end of the expander to thereby axially displace said rear end of the expander so as to facilitate its expansion and contraction.

3. A catheter assembly according to Claim 1, wherein the expander is normally biased to spontaneously expand over the tube, where its fore end and its rear end are affixed over the tube; and whereby the manipulating sleeve extends over the expander and retains it at its retracted configuration whereby upon retraction of the sleeve the expander is gradually exposed to thereby expand its exposed portion.

4. A catheter assembly according to Claim 2, whereby the sleeve is pushed forwards to expand the expander.

5. A catheter assembly according to Claim 2, wherein the front end of the sleeve is articulated to the rear end of the expander.

6. A catheter assembly according to Claim 1, wherein the expander is a wire mesh.

7. A catheter assembly according to Claim 1, wherein the expander has an effective area sufficient to expand a heart valve.

8. A catheter assembly according to Claim 7, wherein the length of the effective area is of a length sufficient to expand a tricuspid valve leaflets to facilitate blood flows from the right ventricle to the right atrium.

9. A catheter assembly according to Claim 8, wherein the length of the effective area is between about 1 cm to 12 cm.

10. A catheter assembly according to Claim 1, wherein the third pressure conduit is formed within a gap between the tube and the sleeve, and the inlet port displaces with the sleeve.

11. A catheter assembly according to Claim 2, wherein a sealing ring is fitted over the sleeve adjacent the rear end of the expander to prevent fluid passage through the expander into the gap.

12. A catheter assembly according to Claim 11, wherein an inlet port of the third pressure conduit is formed adjacent the front end of the sleeve and behind the sealing ring, to facilitate fluid pressure pick-up through the third pressure conduit.

13. A catheter assembly according to Claim 1, wherein the first pressure point is obtained in a pulmonary artery, the second pressure point is obtained in a right ventricle and the third pressure point is obtained in a right atrium.

## Patentansprüche

1. Katheteranordnung, umfassend:
- einen Katheterschlauch (12) mit einem proximalen Ende (16) und einem distalen Ende (14), die ausgebildet sind, axial ein venöses System und den rechten Ventrikel zu durchlaufen;
- wobei an das distalen Ende ein Navigationsballon (18) gepasst ist, der durch ein Aufblaslumen (19) aufblasbar ist, das sich teilweise durch einen Großteil des Schlauchs erstreckt;
- ein Ausdehnungselement (20), das einen Abschnitt des Katheters umgibt, sich zwischen einem hinteren Ende und einem vorderen Ende erstreckt und einen Blutfließweg aufweist;
- eine Betätigungshülse (30) mit einer Stirnseite und einem hinteren Ende, die gleitfähig einen Abschnitt des Schlauchs umhüllt, die sich von dem Ausdehnungselement nach hinten erstreckt, und einen Zwischenraum zwischen dem Schlauch und der Hülse bildet, wodurch das Ausdehnungselement angepasst ist, sich durch Betätigung der Hülse radial auszudehnen und zusammenzuziehen;
- wobei der Schlauch ferner ein erstes Drucklumen (26) umfasst, das sich von dem distalen Ende erstreckt, zum Messen eines ersten Druckpunktes durch eine erste Drucköffnung (27);
ein zweites Drucklumen (28), das sich von dem vor dem vorderen Ende des Ausdehnungselementes Angrenzenden erstreckt, um einen zweiten Druckpunkt durch eine zweiten Drucköffnung (29) zu messen; und
eine dritte Druckleitung (32), die sich von dem dem hinteren Ende des Ausdehnungselementes Angrenzenden erstreckt, um einen dritten Druckpunkt durch eine Einlassöffnung (36) zu messen;
wobei die ersten und zweiten Drucklumen und die dritte Druckleitung an den proximalen Enden der Schläuche an Druckablasskupplungen enden.

2. Katheteranordnung nach Anspruch 1, wobei das Ausdehnungselement sich normalerweise glatt über den Schlauch erstreckt und wobei dessen vorderes Ende über den Schlauch befestigt ist und sein hinteres Ende gleitend darauf veschiebbar ist; und wobei eine Stirnseite der Betätigungshülse mit einem hinteren Ende des Ausdehnungselements in Eingriff steht, um somit das hintere Ende des Ausdehnungselements axial zu verschieben, um so dessen Ausdehnung und Zusammenziehen zu ermöglichen.

3. Katheteranordnung nach Anspruch 1, wobei das Ausdehnungselement normalerweise vorgespannt ist, um sich spontan über dem Schlauch auszudehnen, wobei sein vorderes Ende und sein hinteres Ende über dem Schlauch befestigt sind; und wobei sich die Betätigungshülse über das Ausdehnungselement erstreckt und es in seinem zurückgezogenen Zustand hält, wodurch beim Zurückziehen der Hülse das Ausdehnungselement allmählich freigelegt wird, um **dadurch** seinen freigelegten Abschnitt auszudehnen.

4. Katheteranordnung nach Anspruch 2, wobei die Hülse nach vorne geschoben wird, um das Ausdehnungselement auszudehnen.

5. Katheteranordnung nach Anspruch 2, wobei die Stirnseite der Hülse an dem hinteren Ende des Ausdehnungselements angelenkt ist.

6. Katheteranordnung nach Anspruch 1, wobei das Ausdehnungselement ein Drahtgitter ist.

7. Katheteranordnung nach Anspruch 1, wobei das Ausdehnungselement eine Wirkfläche aufweist, die zum Ausdehnen einer Herzklappe ausreichend ist.

8. Katheteranordnung nach Anspruch 7, wobei die Länge der Wirkfläche eine Länge aufweist, die zum Ausdehnen eines Trikuspidalklappensegels ausreichend ist, um Blutflüsse von dem rechten Ventrikel zum rechten Atrium zu ermöglichen.

9. Katheteranordnung nach Anspruch 8, wobei die Länge der Wirkfläche zwischen ungefähr 1 cm bis 12 cm liegt.

10. Katheteranordnung nach Anspruch 1, wobei die dritte Druckleitung innerhalb eines Zwischenraums zwischen dem Schlauch und der Hülse gebildet ist, und die Einlassöffnung verschiebt sich mit der Hülse.

11. Katheteranordnung nach Anspruch 2, wobei ein Dichtring über die Hülse angrenzend an das hinteren Ende des Ausdehnungselements gepasst wird, um Flüssigkeitsdurchlauf durch das Ausdehnungselement in den Zwischenraum zu verhindern.

12. Katheteranordnung nach Anspruch 11, wobei eine
Einlassöffnung der dritten Druckleitung angrenzend an die Stirnseite der Hülse und hinter dem Dichtring gebildet ist, um Flüssigkeitsdruckaufnahme durch die dritte Druckleitung zu ermöglichen.

13. Katheteranordnung nach Anspruch 1, wobei der erste
Druckpunkt in einer Lungenarterie erhalten wird, der zweite Druckpunkt in einem rechten Ventrikel erhalten wird, und der dritte Druckpunkt in einem rechten Atrium erhalten wird.

## Revendications

1. Assemblage cathéter comprenant :
un tube de cathéter (12) ayant une extrémité proximale (16) et une extrémité distale (14), configuré pour passer de manière axiale dans un système veineux et le ventricule droit ;
ladite extrémité distale étant équipée d'un ballonnet de navigation (18) pouvant être gonflé par le biais d'une lumière de gonflage (19) s'étendant partiellement dans une majeure partie du tube ;
un expanseur (20) entourant une section dudit cathéter, s'étendant entre une extrémité arrière et une extrémité avant, et ayant une trajectoire d'écoulement de sang ;
un manchon de manipulation (30) ayant une extrémité avant et une extrémité arrière enveloppant de manière coulissante une partie du tube s'étendant vers l'arrière à partir de l'expanseur et formant un espace entre le tube et ledit manchon, moyennant quoi ledit expanseur est adapté pour s'expanser radialement et se contracter par le biais de la manipulation du manchon ;
le tube comprenant en outre une première lumière de pression (26) s'étendant à partir de l'extrémité distale pour mesurer un premier point de pression par un premier orifice de pression (27) ;
une seconde lumière de pression (28) s'étendant à partir de la partie adjacente en face de l'extrémité avant de l'expanseur pour mesurer un deuxième point de pression par un second orifice de pression (29) ; et
un troisième conduit de pression (32) s'étendant à partir de la partie adjacente à l'extrémité arrière de l'expanseur pour mesurer un troisième point de pression par un orifice d'entrée (36) ;
lesdites première et seconde lumières de pression et le troisième conduit de pression se terminent à l'extrémité proximale des tubes au niveau des couplages de sortie de pression.

2. Assemblage cathéter selon la revendication 1, dans lequel l'expanseur se prolonge normalement à affleurement au-dessus du tube et où son extrémité avant est fixée sur le tube et son extrémité arrière peut être déplacée de manière coulissante sur celui-ci ; et où une extrémité avant du manchon de manipulation est mise en prise avec une extrémité arrière de l'expanseur pour déplacer ainsi de manière axiale ladite extrémité arrière de l'expanseur afin de faciliter son expansion et sa contraction.

3. Assemblage cathéter selon la revendication 1, dans lequel l'expanseur est normalement sollicité pour s'expanser spontanément sur le tube, où son extrémité avant et son extrémité arrière sont fixées sur le tube ; et moyennant quoi le manchon de manipulation s'étend sur l'expanseur et le retient dans sa configuration rétractée moyennant quoi suite à la rétraction du manchon, l'expanseur est progressivement exposé pour expanser ainsi sa partie exposée.

4. Assemblage cathéter selon la revendication 2, moyennant quoi le manchon est poussé vers l'avant pour expanser l'expanseur.

5. Assemblage cathéter selon la revendication 2, dans lequel l'extrémité avant du manchon est articulée par rapport à l'extrémité arrière de l'expanseur.

6. Assemblage cathéter selon la revendication 1, dans lequel l'expanseur est un treillis métallique.

7. Assemblage cathéter selon la revendication 1, dans lequel l'expanseur a une surface effective suffisante pour expanser une valvule cardiaque.

8. Assemblage cathéter selon la revendication 7, dans lequel la longueur de la surface effective est d'une longueur suffisante pour expanser une valvule tricuspide afin de faciliter l'écoulement de sang du ventricule droit à l'oreillette droite.

9. Assemblage cathéter selon la revendication 8, dans lequel la longueur de la surface effective est comprise entre environ 1 cm à 12 cm.

10. Assemblage cathéter selon la revendication 1, dans lequel le troisième conduit de pression est formé à l'intérieur d'un espace entre le tube et le manchon, et l'orifice d'entrée se déplace avec le manchon.

11. Assemblage cathéter selon la revendication 2, dans lequel une bague d'étanchéité est montée sur le manchon adjacent à l'extrémité arrière de l'expanseur pour empêcher le passage du fluide par l'expanseur, dans l'espace.

12. Assemblage cathéter selon la revendication 11, dans lequel un orifice d'entrée du troisième conduit de pression est formé de manière adjacente à l'extrémité avant du manchon et derrière la bague d'étanchéité, pour faciliter la détection de la pression de fluide à travers le troisième conduit de pression.

13. Assemblage cathéter selon la revendication 1, dans lequel le premier point de pression est obtenu dans une artère pulmonaire, le deuxième point de pression est obtenu dans un ventricule droit et le troisième point de pression est obtenu dans une oreillette droite.
